(19) Europäisches Patentamt
European Patent Office
Office européen des brevets

(11) Publication number: **0 216 604 B1**

# EUROPEAN PATENT SPECIFICATION

(45) Date of publication of patent specification:
**16.01.91 Bulletin 91/03**

(51) Int. Cl.$^5$: **C07C 1/20, C10G 3/00**

(21) Application number: **86307184.1**

(22) Date of filing: **18.09.86**

(54) Process for converting oxygenates into alkylated liquid hydrocarbons.

(30) Priority: **23.09.85 US 779347**
**23.09.85 US 779367**
**23.09.85 US 779369**
**23.09.85 US 779363**
**23.09.85 US 779373**

(43) Date of publication of application:
**01.04.87 Bulletin 87/14**

(45) Publication of the grant of the patent:
**16.01.91 Bulletin 91/03**

(84) Designated Contracting States:
**BE DE FR GB IT NL SE**

(56) References cited:
**DE-A- 3 032 658**
**US-A- 4 367 356**
**US-A- 4 450 311**
**US-A- 4 506 106**

(73) Proprietor: **MOBIL OIL CORPORATION**
**150 East 42nd Street**
**New York New York 10017 (US)**

(72) Inventor: **Gould, Ronald Michael**
**223 Champion Way**
**Sewell New Jersey 08080 (US)**
Inventor: **Tabak, Samuel Allen**
**204 East Pine Street**
**Wenonah New Jersey 08090 (US)**
Inventor: **Owen, Hartley**
**5 Riverview Terrace**
**Belle Meade New Jersey 08502 (US)**
Inventor: **Wright, Bernard Stanley**
**13 Shagbark Lane**
**East Windsor New Jersey 08520 (US)**

(74) Representative: **Cooper, John Anthony et al**
**Mobil Court 3 Clements Inn**
**London WC2A 2EB (GB)**

## Description

This invention relates to a process for converting oxygenates, such as methanol and dimethyl ether (DME), into liquid hydrocarbons, and more particularly, to a process for converting the oxygenate feedstock catalytically into an intermediate lower olefinic stream and alkylating isobutane and other isoparaffins with olefins to produce light distillate and/or alkylate gasoline.

In order to provide an adequate supply of liquid hydrocarbons for use as synfuels or chemical feedstocks, various processes have been developed for converting coal and natural gas into gasoline and distillate. A substantial body of technology has grown to provide oxygenated intermediates, especially methanol. Large scale plants can convert methanol and similar aliphatic oxygenates into liquid fuels, especially gasoline. However, the demand for a broader range of hydrocarbons has led to the development of processes for making high octane gasoline and quality diesel fuel from $C_3^+$ olefins.

Recent developments in zeolite catalysts and hydrocarbon conversion processes have created interest in utilizing olefinic feedstocks for producing $C_5^+$ gasoline and diesel fuel, for example. In addition to the basic work derived from ZSM-5 type zeolite catalysts, a number of discoveries have contributed to the development of new industrial processes.

Catalysts comprising medium pore ZSM-5 type zeolites are useful for converting methanol and other lower aliphatic alcohols and corresponding ethers into olefins. Particular interest has been directed to a catalytic process for converting low cost methanol into valuable hydrocarbons rich in ethene and $C_3^+$ alkenes. Various processes of this type are described in U.S. Patents 3,894,107, 3,928,483, 4,025,571, 4,423,274 and 4,433,189. It is generally known that the process for converting methanol to olefins can be optimized to produce a major fraction of $C_2$-$C_4$ olefins. Prior process proposals have included a separation section to recover

Prior process proposals have included a separation section to recover ethene and other gases from byproduct water and $C_5$+ hydrocarbon liquids.

US-A-4 506 106 describes an integrated process for converting oxygenates to heavy hydrocarbon products. The process comprises contacting the feed with a zeolite catalyst to convert a portion of the feed to $C_2$-$C_4$ olefins and a minor amount of $C_5$-heavy hydrocarbon ; converting the effluent light hydrocarbon stream by selective sorption and contacting the sorbate with an oligomerization catalyst to yield a distillate stream.

US-A-4 367 356 discloses a process for the production of gasoline from $C_4$ hydrocarbon feedstock. The feedstock is split into two streams, one for dimerization and the other, alkylation.

The present invention is based on the observation that methanol, DME or the like may be converted into liquid fuels, particularly gasoline, aliphatic distillate and alkylate, in a multi-stage process with integration between the major process stages providing an ethene-rich recycle stream, and a $C_5$+ liquid recycle stream as sorbent and an alkylate product stream. The hydrocarbon effluent stream from the initial oxygenate conversion step, after byproduct hydrocarbon and water removal, compression, separation, sorption and fractionation can be fed to an alkylation stage for conversion to heavier hydrocarbons. Ethene may be recovered by interstage separation and recycled. The recycled ethene is reaction with methanol/DME and other oxygenates in the presence of ZSM-5 type zeolite catalysts and hence may be recycled substantially to extinction.

In accordance with its broadest aspect, the present invention provides an integrated continuous process for converting oxygenated organic feedstock to liquid hydrocarbons comprising the steps of

(a) contacting feedstock with zeolite catalyst in a primary catalyst stage at elevated temperature and moderate pressure to convert at least a portion of the feedstock oxygenate to hydrocarbons containing a major fraction of $C_2$-$C_4$ olefins and a minor fraction containing $C_5$+ hydrocarbons ; (b) cooling and separating effluent from step (a) to provide an aqueous liquid byproduct stream, a heavy hydrocarbon liquid stream and a light hydrocarbon vapor stream rich in $C_2$-$C_4$ olefins ; (c) compressing at least a portion of the olefinic light hydrocarbon stream to condense a liquid olefinic hydrocarbon stream rich in $C_3$-$C_4$ olefins and recovering an ethene-rich gaseous stream ; (d) further reacting the condensed liquid olefinic hydrocarbon stream from step (c) with isobutane in a secondary alkylation stage with acid catalyst to convert at least a portion of $C_3$-$C_4$ olefins to a heavier $C_7$+ liquid hydrocarbon product stream comprising alkylate gasoline ; and (e) recycling ethene in a gaseous stream to the primary catalytic stage.

According to a particular aspect of the process of the invention, the feedstock is converted into liquid hydrocarbons by the steps of (a) contacting the feedstock with a zeolite catalyst to obtain a product containing an olefinic fraction rich in $C_2$-$C_4$ olefins and a minor fraction containing $C_5$+ aliphatic and aromatic hydrocarbons ; (b) cooling the effluent from step (a) and separating the cooled effluent to obtain an aqueous liquid stream, a heavy hydrocarbon liquid stream and a light hydrocarbon vapor stream rich in $C_2$-$C_4$ olefins;

(c) compressing the light hydrocarbon vapor stream from step (b) and contacting it with a liquid hydrocarbon sorbent stream containing a major amount of heavy hydrocarbon liquid stream from step (b) to selectively sorb $C_3+$ components into the liquid sorbate stream ; (d) fractionating the sorbate from step (c) to obtain a gasoline-rich stream and a light hydrocarbon stream rich in $C_3$-$C_4$ olefins ; (e) reacting the olefinic light hydrocarbon stream from step (d) with isoparaffin in the presence of an acid catalyst under alkylation conditions to convert at least a portion of $C_3$-$C_4$ olefins into a $C_7+$ liquid hydrocarbon product stream comprising alkylate gasoline ; (f) recycling ethene from step (c) to step (a) ; and (g) recycling a portion of gasoline from step (d) to step (c) as lean sorbent.

According to a further particular aspect of the process of the invention, the feedstock is converted into liquid hydrocarbons by the steps of (a) fractionating the $C_2$-$C_4$ olefins to obtain a stream rich in ethene and a stream containing $C_3+$ olefins ; (b) contacting a major portion of the $C_3+$ stream from step (a) with an oligomerization catalyst comprising a shape-selective, medium-pore zeolite at an elevated temperature and pressure to obtain a heavier hydrocarbon effluent stream comprising distillate, gasoline and lighter hydrocarbons ; (c) fractionating the effluent from step (b) to obtain distillate, gasoline and a $C_3$-$C_4$ hydrocarbon stream containing isobutane ; (d) reacting isobutane from step (c) with a second portion of $C_3+$ olefin from step (a) in the presence of an acid catalyst under alkylation conditions to produce $C_7+$ alkylate.

Advantageously, the first stage catalyst comprises ZSM-5 type zeolite. Preferably, ethene is recycled to the first stage in an amount of about 1 to 20 parts ethene per 100 parts by weight of methanol equivalent in the feedstock. By fractionating the gaseous effluent separated from the first stage effluent, a recycle gas stream may be recovered containing at least 90% of ethene from the first catalyst stage, and an olefinic stream rich in $C^+_3$ olefins, especially propene and butylenes, is provided for reaction with various isoparaffins, such as isobutane.

Numerous oxygenated organic compounds may be contained in the feedstock passed to the first catalyst stage. Since methanol and its ether derivative (DME) are industrial commodities readily available from synthesis gas or the like, these materials are preferred starting materials. It is to be understood, however, that the process can employ methanol, dimethylether and mixtures thereof, as well as other aliphatic alcohols, ethers, ketones and/or aldehydes. It is known that oxygenates can be partially converted by dehydration, as in the catalytic reaction of methanol over gamma-alumina to produce DME. Typically, an equilibrium mixture of methanol, DME and water is produced by partial dehydration. This reaction takes place in either conversion of methanol to lower olefins or methanol to gasoline.

The preferred catalyst comprises a crystalline zeolite having a silica to alumina ratio of at least 12, a constraint index of 1 to 12 and acid cracking activity of 1-200. Acid cracking activity values of 1-50 are preferred for olefin conversion, and of 50-200 are preferred for olefin oligomerization. Representative of such ZSM-5 type zeolites are ZSM-5, ZSM-11, ZSM-12, ZSM-22, ZSM-23, ZSM-35, ZSM-38 and ZSM-48. ZSM-5 is described in U.S. Patent 3,702,886 and U.S. Patent Re. 29,948 ; ZSM-11 is described in U.S. Patent 3,709,979. ZSM-12 is described in U.S. Patent 3,832,449 ; ZSM-23 is described in U.S. Patent 4,076,842; ZSM-35 is described in U.S. Patent 4,016,245 ; and ZSM-38 is described in U.S. Patent 4,046,839. An especially suitable catalyst for oxygenate conversion comprises HZSM-5 zeolite and an alumina binder.

Other catalysts and processes suitable for converting methanol/DME to lower olefins are described in U.S. Patents 4,393,265 and U.S. 4,387,263 and European Patent Application 0081683. Another suitable zeolite is ZSM-45. In addition to the preferred alumino-silicates, the equivalent borosilicate and ferrosilicate materials may be employed. ZSM-5 type zeolites are particularly advantageous because the same material may be employed for dehydration of methanol to DME, conversion to lower olefins and oligomerization.

The invention will now be described in greater detail by way of example only with reference to the accompanying drawings, in which

FIG. 1 is a flow sheet for a first process according to the invention, showing the major unit operations and process streams ;

FIG. 2 is a schematic representation of a preferred inter-stage separation system for ethene recovery in accordance with the first process ;

FIG. 3 is a schematic representation of an alternative separation system according to the first process;

FIG. 4 is a flow sheet for a second process according to the invention showing the major unit operations and process streams ;

FIG. 5 is a flow sheet for a third process according to the invention showing the major unit operations and process streams ;

FIG. 6 is a schematic representation of a preferred inter-stage separation system for ethene recovery according to the second and third processes ;

FIG. 7 is an alternative flow sheet depicting sorption, fractionation and alkylation units schematically, according to the second process ;

FIG. 8 is an alternative flow sheet depicting sorption, fractionation and alkylation units schematically, according to the third process.

FIG. 9 is a flow sheet for a fourth process according to the invention showing the major unit operations and process streams ;

FIG. 10 is a schematic representation of an alternative inter-stage separation system according to the fourth process ;

FIG. 11 is a flow sheet for a fifth process according to the invention showing the major unit operations and process streams ;

FIG. 12 is a schematic representation of a preferred inter-stage separation system according to the fifth process ; and

FIG. 13 is an alternative flow sheet for the fifth process.

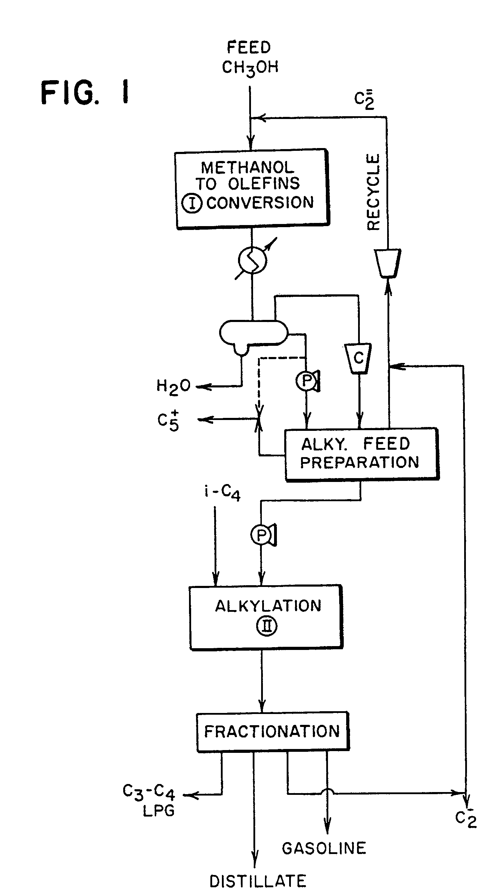

Referring first to FIG. 1 of the drawings, the process feedstock (methanol and/or DME, for example) is fed to the first conversion stage (I) where it is converted into lower olefins and gasoline hydrocarbons plus water by dehydration. Byproduct water is recovered by simple phase separation of the cooled effluent. Liquid hydrocarbons consisting essentially of $C_5^+$ gasoline boiling range materials may be recovered or pumped to the higher second stage pressure. At least a portion of the vapor phase effluent from the first stage is compressed to alkylation pressure, and reacted at high pressure and moderate temperature in contact with an acidic alkylation catalyst. Second stage (II) effluent is then separated into light gases for recycle in part, $C_7^+$ gasoline and/or distillate range hydrocarbons.

Alkylation of ethene with methanol over a ZSM-5 zeolite catalyst has been described by Kaeding et al (J. Catalysis ; Jan. 1980, Aug. 1984), and it is known to recycle ethene in the production of aromatic gasoline from methanol over zeolites (U.S. Patent 3,998,899). In a fluidized bed plant for converting methanol into lower olefins and gasoline, recycle of ethene at a rate of 2.5 parts by weight per 100 parts $CH_2$ equivalent in the feedstock methanol provides a product yield that is substantially the same, as shown in Table I. These continuous runs are conducted under the same conditions.

## TABLE I

### Hydrocarbon Product Yield, Wt %

| Component | Without Recycle | With Ethene Recycle |
|---|---|---|
| $C_1$ | 0.8 | 0.8 |
| $C_2$ | 0.3 | 0.3 |
| $C_2^=$ | 2.5 | 2.7 |
| $C_3$ | 4.4 | 4.5 |
| $C_3^=$ | 4.6 | 4.5 |
| $nC_4$ | 2.1 | 2.1 |
| $iC_4$ | 10.8 | 10.4 |
| $C_4^=$ | 5.4 | 5.1 |
| $C_5^+$(Gasoline) | 69.1 | 69.6 |
| Total | 100.0 | 100.0 |

$T = 407°C$, $P = 400KPa$, $WHSV = 2.65$ (based on HZSM-5).

The process may be optimized by employing fluid bed first stage conditions at a temperature of 425 to 550°C, a pressure of 100 to 800 kPa and weight hourly space velocity of 0.5 to 3.0 based on ZSM-5 and methanol equivalent in the first stage feedstock. Suitable fluid bed equipment and operating conditions are described in U.S. Patent 4,547,616 ; fixed bed MTO systems are described in U.S. Patent 4,542,252.

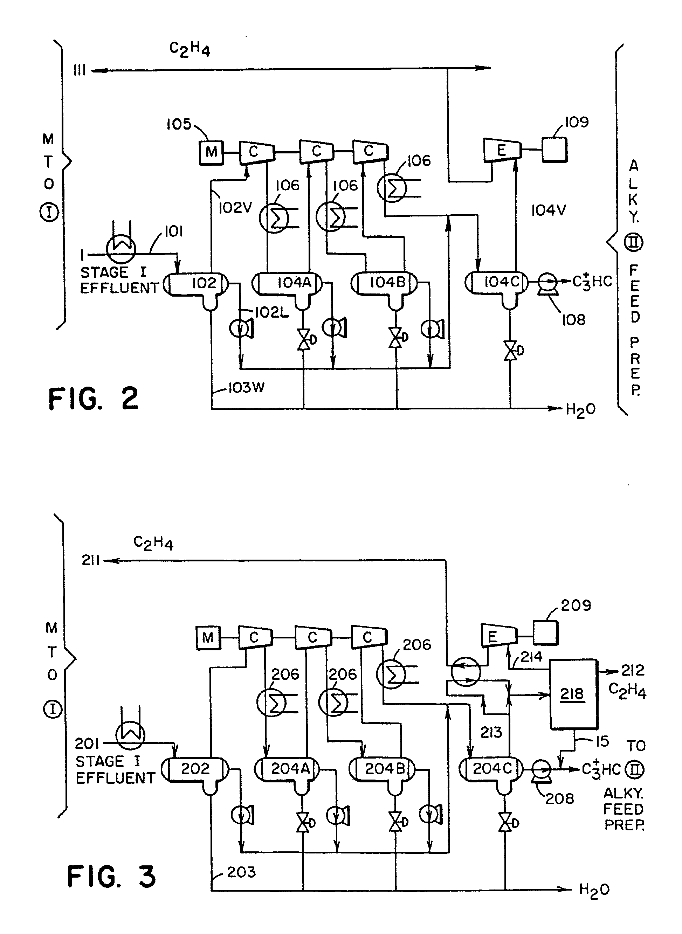

Referring now to FIG. 2, the light hydrocarbon vapor stream separated from the first stage effluent is compressed in a plurality of compression stages to condense liquid olefinic hydrocarbons (HC).The full reaction effluent of the first stage plant is passed via conduit 101 and first phase separator 102 to provide a first vapor stream 102V, rich in $C_4$-hydrocarbons, liquid hydrocarbons stream 102L, and by product water stream 103W. The liquid (eg-$C_5^+$) stream 102L is combined with a corresponding liquid HC from succeeding separators and withdrawn. The primary vapor stream 102V is adiabatically compressed by multi-stage motor-compressor set 105, cooled via exchanger 106 and passed to a succeeding separator 104A, at which point the preceeding phase separation technique is repeated. Similarly other separators 104B and 104C operate to provide an ethene-rich recycle stream 104V, which is passed to turbo-expander 109E and thus at original pressure back via line 111 to the olefins production in the first stage. Advantageously, the first stage effluent is received at about atmospheric pressure (for example 100-150 kPa) and compressed in plural stages to a pressure of about 1100-3500 kPa and separated in the final vessel 104C at about ambient temperature (20-60°C). Olefinic liquids rich in $C_3^+$ aliphatic hydrocarbons are recovered from the final compressor stage via pump 108 which passes the liquid HC stream to the following second stage alkylation unit.

A further modification of the interstage ethene separation technique described above is depicted in the flow diagram in FIG. 3, in which corresponding apparatus and process streams are identified by corresponding numbers to those of FIG. 2. In this modification, ethene-rich vapor withdrawn from the last separator 204C via line 213 is cooled by heat exchange and further processed to increase ethene purity in ethene unit 218. It will be understood that ethene can be treated in a cryogenic plant cold box, de-ethanizer tower, absorption unit or the like to remove undesirable components prior to recycle 211 and/or recovery 212. A suitable selective sorption unit is described in U.S. Patent 4,471,147. Preferably, compressed light hydrocarbons are fractionated to recover a recycle stream containing at least 90 mole percent ethene. This can be achieved by selectively absorbing $C_3^+$ components in a $C_5^+$ liquid hydrocarbon sorbent stream.

The second stage alkylation process is a well known industrial technique for reacting alkenes with tertiary alkanes (isoparaffins), such as isobutane, isopentane, and isohexane, for example. The resulting product is a $C_7^+$ branched chain paraffinic material useful as aviation gasoline and jet fuel, for example. The alkylation of paraffins can be carried out either thermally or catalytically ; however, acid catalysis is preferred. Thermal or noncatalytic alkylation of a paraffin with an olefin is carried out at high temperatures (about 500°C) and pressures 21-41 MPa. Under these conditions, both normal and isoparaffins can be brought into reaction by a free-radical mechanism. Thermal alkylation is not known to be practiced commercially.

The catalytic alkylation of paraffins involves the addition of an isoparaffin containing a tertiary hydrogen atom to an olefin. The process is used in the petroleum industry to prepare highly branched paraffins mainly in the $C_7$ to $C_9$ range, that are high-quality fuels. The overall process is complex, requiring control of operating conditions and of catalyst. The process conditions and the product composition depend on the particular hydrocarbons involved.

The preferred processes are those brought about by the conventional protonic and Lewis catalysts. Propene can be brought into reaction with an isoparaffin in the presence of either concentrated sulfuric acid or hydrogen fluoride. The heptanes produced by alkylation of isobutane with propene are mainly 2,3- and 2,4-dimethylpentanes. Propene is alkylated preferably as a component of a $C_3$-$C_4$ fraction. HF catalysts for alkylation of isobutane with 1- and 2-butenes give both dimethylhexanes and trimethyl-pentanes. The product obtained from alkylation of isobutane with isobutylene at low temperature (for example –25°C) with hydrogen fluoride is 2,2,4-trimethylpentane.

During use the acid catalysts may become diluted with byproduct hydrocarbons and as a result decrease in activity. Sulfuric acid concentrations should be maintained at about 90%. Hydrogen fluoride concentrations of 80-90% are common, although the optimum concentration depends on the reaction temperature and reactor geometry. Operation below these acid concentrations generally causes incomplete conversion or polymerization. With sulfuric acid, the product quality is improved when temperatures are reduced to the range of 0-10°C. Cooling requirements are obtained by low temperature flashing of unreacted isobutane. With hydrogen fluoride, the process is less sensitive to temperature, and temperatures of 0-40°C can be used. Some form of heat removal is essential because the heat of reaction is approximatley $14 \times 10^5$ J/kg of butenes. Typically, the elevated pressure for alkylation by these acid catalysts is 1500 to 3000 kPa.

In order to prevent polymerization of the olefin charge, an excess of isobutane is present in the reaction zone. Isobutane-to-olefin molar ratios of 6 :1 to 14 :1 are common, more effective suppression of side reactions being obtained at the higher ratios.

The typical alkylation reaction employs a two-phase system with a low solubility of the isobutane in the catalyst phase. In order to ensure intimate contact of reactants and catalyst, efficient mixing is provided. This is important with sulfuric acid because of the low solubility of isobutane in the catalyst phase. In addition, the higher viscosity of the sulfuric acid requires a greater mixing energy to assure good contact. The solubility of the hydrocarbon reactants in the catalyst phase is increased by the presence of the unsaturated organic diluent held by the acid catalyst. This organic diluent also has been considered a source of carbonium ions that promote the alkylation reaction.

For the hydrofluoric acid system, reactive $i$-$C_4H_8$ readily alkylates to give an excellent product. The alkylation of pure 1-$C_4H_8$ by itself proceeds with considerable isomerization of the 1-$C_4H_8$ to 2-$C_4H_8$ followed by alkylation to give a highly branched product. The presence of $i$-$C_4H_8$ accelerates the alkylation reaction and allows less time for olefin isomerization. Consequently the reaction produces an alkylate with a lowered antiknock value. For the sulfuric acid system, $i$-$C_4H_8$ tends to oligomerize and causes other side reaction products of inferior quality ; but the isomerization of 1-$C_4H_8$ to 2-$C_4H_8$ proceeds more completely, thereby favoring formation of superior products. Thus for mixed olefin feeds such as described above, the two factors

5

with both catalyst systems counteract each other to provide products of similar antiknock properties.

The olefin-producing process may simultaneously generate isobutane, but the amount may be insufficient to alkylate the coproduced olefins. A suitable outside source of isobutane is natural gas or a byproduct of methanol-to-gasoline (MTG) processes.

Suitable alkylation processes are described in U.S. Patents 3,879,489, 4,115,471, and 4,377,721 and in the Kirk-Othmer Encyclopedia of Chemical Technology, Vol. 2, pp. 50-58 (3rd Ed., 1978) John Wiley & Sons.

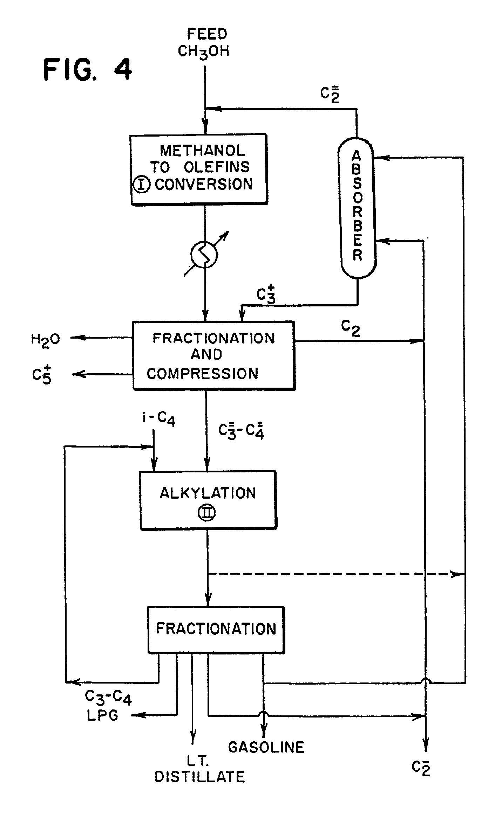

Referring now to FIG. 4, the process feedstock (methanol and/or DME, for example) is fed to the first stage (I) where it is converted into lower olefins and $C_5+$ gasoline hydrocarbon plus water by dehydration. Byproduct water is recovered by simple phase separation from the cooled effluent. Liquid hydrocarbons consisting essentially of $C^+_5$ gasoline boiling range materials may be recovered or pumped to the higher second stage pressure. Vapor phase effluent from the first stage may be compressed to alkylation pressure. Propylene, butylenes and amylenes may be separated from the first stage effluent by sorption fractionation to recover a recycle gas stream containing at least 90% of ethene from the first stage and an olefinic sorbate stream rich in $C_3+$ olefins. A $C_3$-$C_4$ rich olefinic stream may be further prepared for reaction with isobutane or the like at high pressure and low temperature in contact with liquid phase acidic alkylation catalyst. Second stage (II) alkylation effluent is then separated into $C^-_2$ light gases, $C_3$-$C_4$ aliphatics and $C^+_5$ gasoline and/or light distillate boiling range hydrocarbons. Advantageously, isobutane is separated from the second stage effluent for recycle to provide a stoichiometric excess and added with fresh feed ($i$-$C_4$) to the unit. A portion of the liquid alkylate-rich hydrocarbon is recycled to the interstage sorption unit as lean sorbent. The preferred sorption unit is a countercurrent packed tower. Advantageously, the sorbent liquid comprises at least 75 wt% of $C_7$ to $C_9$ isoparaffins which are the alkylate product. This lean sorbent has excellent properties for selective sorption of the propene, butylene and $C_3+$ paraffinic components of the first stage light hydrocarbons. Unfractionated liquid alkylation effluent may be recycled in part, as depicted by the broken line.

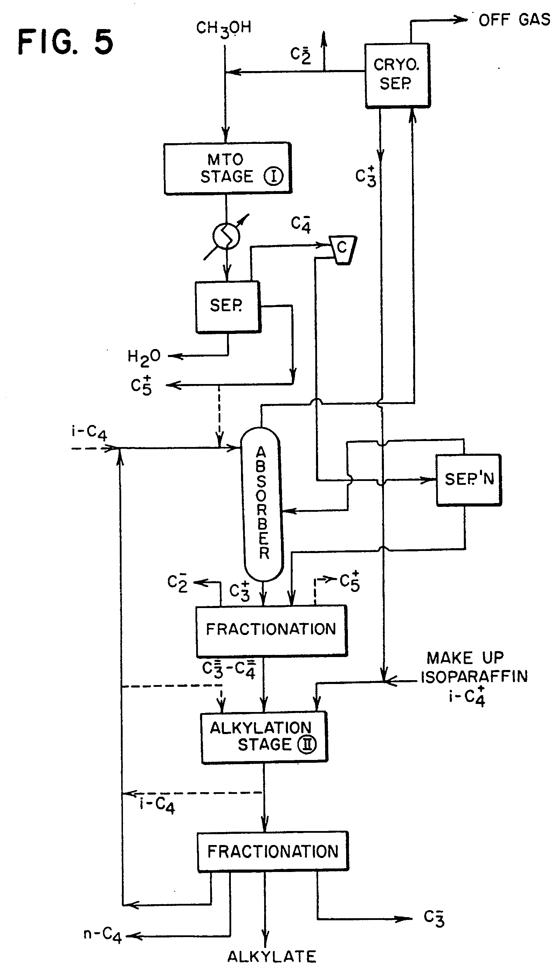

The flow scheme shown in FIG. 5 is generally similar to that shown in FIG. 4 and described above, except that the second stage (II) alkylation effluent is separated into $C^-_3$ light gases, -$C_4$ aliphatics and $C^+_5$ gasoline and/or light distillate boiling range hydrocarbons. Advantageously, isobutane is separated from the second stage effluent for recycle to provide a stoichio-metric excess. Isoparaffin may be added as fresh (makeup) feed directly to the alkylation unit or introduced as lean sorbent unit. A portion of the liquid $C_4+$ isoparaffin-rich hydrocarbon is recycled to the interstage sorption unit as lean sorbent. The preferred sorption unit is a countercurrent packed tower. Advantageously, the sorbent liquid comprises at least 50 wt% of $C_4$ to $C_8$ isoparaffins, especially the reactive tertiary alkanes such as $i$-$C_4$. This lean sorbent has excellent properties for selective sorption of the propene, butylene and $C_3+$ paraffinic components of the first stage light hydrocarbons. Unfractionated liquid alkylation effluent may be recycled in part, as depicted by the broken lines.

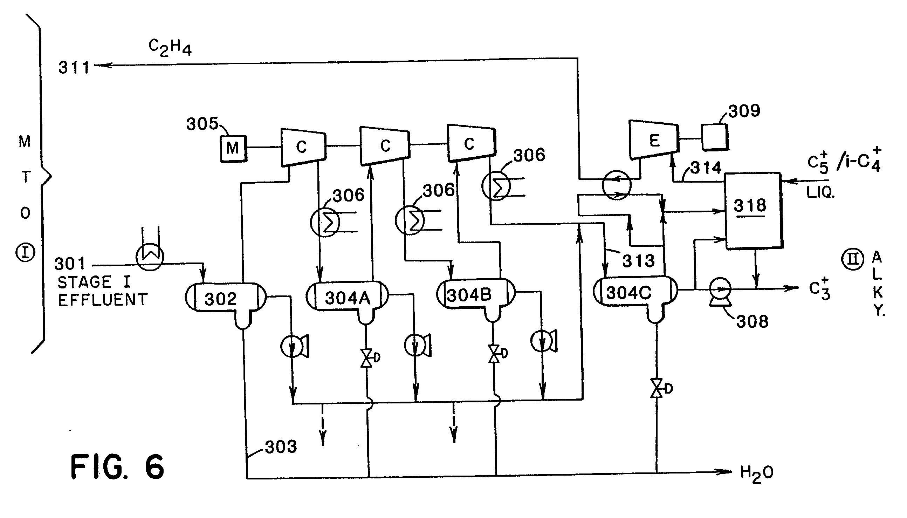

The separation system shown in FIG. 6 is similar to that described above with reference to FIG. 3, although in this case, compressed light hydrocarbons are fractionated by sorption to recover a recycle stream containing at least 90 mole percent ethene. This can be achieved by selectively absorbing $C_3+$ components in a $C^+_5$ liquid hydrocarbon sorbent stream, especially $C_7$ to $C_9$ alkylate.

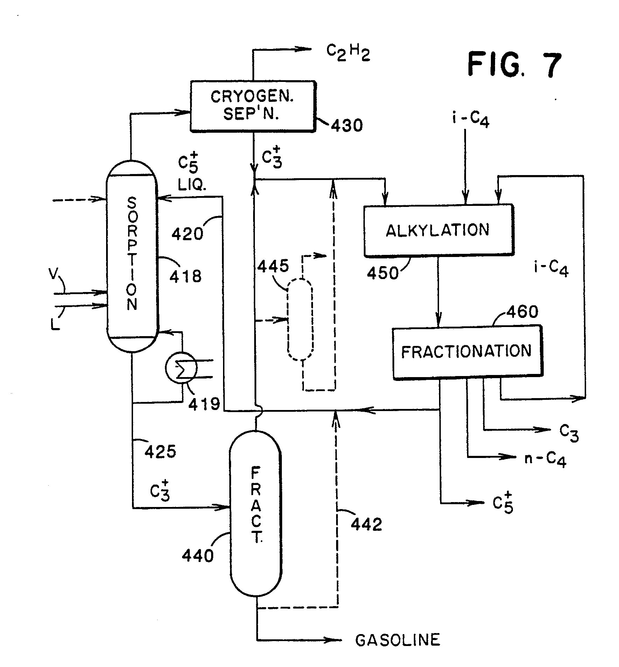

Interstage fractionation can be modified to provide for recovery of purified ethylene and heavier hydrocarbon liquids. In FIG. 7, a sorption fractionator unit 418, comprising a vertical countercurrent contact tower, is equipped with a bottom reboiler 419. Sorbent liquid, rich in $C_7$ to $C_9$ alkylate, is introduced via conduit 420. Optionally, other $C_5+$ liquids may be employed to supplement the alkylate stream. A light olefin feedstream containing $C_2$-$C_4$ alkenes is introduced via vapor conduit V and liquid conduit L. The sorber overhead may contain 40% or more ethene, which can be further purified by cryogenic separation unit 430 to remove any remaining $C_3+$ hydrocarbons and thus recover pure ethene. The sorbate stream 425, rich in $C_3$-$C_4$ alkenes, is fractionated in distillation tower 440 to recover gasoline boiling range hydrocarbons, and this tower may be operated to obtain a heavy $C_6$ product, with $C_5$ components passing either overhead or with the bottoms. Optionally, this overhead fraction is de-ethanized by tower 445, and the liquified sorbate fraction, rich in $C_3$-$C_4$ components is combined with $C_3+$ components from the cryogenic separation unit and fed at high pressure (for example up to 3000 kPa) to alkylation reactor 450, where it is contacted with an isoparaffin, such as isobutane ($i$-$C_4$) in the presence of an alkylation catalyst to produce alkylate hydrocarbons. The alkylation fractionation system 460 may be operated in a known manner to separate the reactor effluent into several fractions, including $i$-$C_4$ recycle to provide excess isoparaffin, light gas ($C_3$), normal paraffin ($n$-$C_4$) and $C_5+$ liquid. At least a portion of the $C_5+$ components, especially the $C_7$ to $C_9$ alkylate products, is passed via conduit 420 to the sorption fractionator unit as lean sorbent. Recovered $C_5+$ liquids from fractionation unit 460 may be further refined to recover aviation gasoline and light distillate, for example.

If additional sorbent liquid is required, a bottom fraction from unit 440 may be utilized, as indicated by broken line 442. However, it is preferred to employ sorbent comprising a major amount of $C_7$-$C_9$ hydrocarbons, particularly at least 75% paraffinic alkylate.

The data in Table II represents a material balance and absorber operating conditions for a countercurrent contact tower unit design according to FIG. 7 and Runs 1-3. The vertical tower has 21 theoretical stages, with $C_7$-$C_9$ alkylate (lean oil #1) from second stage product fractionator being introduced at the top (stage 1), heavy liquid separated from the first stage effluent (lean oil #2) being introduced at stage 5, olefin vapor and liquid feed being fed at stages 12 and 13 respectively. Heat of sorption is removed by pump-around cooling at stages 5 and 8. The three runs correspond to different lean oil rates. In the overhead stream, molar units are gm-moles per metric tonne (MT) of methanol (MeOH) charged to the process. The operating conditions are chosen to provide a maximum ethene content of 0.2 mol% in the sorbate.

## TABLE II

## ABSORBER OPERATION

| Run No. | 1 | 2 | 3 |
|---|---|---|---|
| **Material Balance** wt% of MeOH charge | | | |
| (1) C$_6$+ Gasoline | 50.0 | 22.0 | 17.6 |
| (2) Alkylate | 15.3 | 47.3 | 51.7 |
| (3) n-butane | 4.9 | 1.0 | 0.8 |
| (4) propane | 3.9 | 3.8 | 3.8 |
| (5) offgas | 1.5 | 1.5 | 1.6 |
| (6) water | 56.4 | 56.4 | 56.4 |
| (7) i-C$_4$ makeup | −32.0 | −32.0 | −31.9 |
| (8) methanol | −100.0 | −100.0 | −100.0 |
| **Absorber*** | | | |
| Lean Oil #1 (stage 1) g-moles/tonne MeOH | 4.44 | 1.19 | 0.69 |
| Lean Oil #2 (stage 5) g-moles/tonne MeOH | 1.01 | 1.01 | 1.01 |
| TOTAL | 5.45 | 2.20 | 1.70 |
| overhead flow, SCM/ton MeOH | 42.0 | 45.3 | 49.5 |
| propane, g-moles/tonne MeOH | 0.0 | 6.5 | 18.5 |
| propylene | 1.0 | 120.4 | 280.0 |
| n-butane | 3.9 | 18.3 | 18.1 |
| isobutane | 0.1 | 0.6 | 1.5 |
| 1-butylene | 0.0 | 1.0 | 5.4 |
| n-pentanes | 0.7 | 0.6 | 0.6 |
| iso-pentanes | 40.2 | 30.0 | 28.4 |
| pentanes | 0.0 | 0.0 | 0.1 |
| C$_6$+ | 16.5 | 17.4 | 17.9 |
| Overhead pressure, kPa | 2068 | 2068 | 2068 |
| Cooling pump around, MJ/ tonne MeOH | −36.7 | −35.4 | −31.7 |
| Reboiler Duty, MS/tonne MeOH | 206.1 | 106.6 | 94.2 |
| Ethylene recovery in overhead g-moles/ tonne MeOH | 938 | 938 | 938 |
| Mole% purity | 53 | 49 | 45 |

*based on 0.2 mol% C$_2$ in bottoms

Runs 4 to 6 (Table III) are similar to Runs 1 to 3, except that the paraffinic lean oil is alkylation reactor effluent containing 78 mole% isobutane, 6% C$_7$-C$_9$ alkylate and C$_5^-$ hydrocarbons.

## TABLE III

### ABSORBER OPERATION

| Run No. | 4 | 5 | 6 |
|---|---|---|---|

**Material Balance**
wt% of MeOH charge

| | 4 | 5 | 6 |
|---|---|---|---|
| (1) $C_6$+ Gasoline | 15.0 | 12.3 | 11.9. |
| (2) Alkylate | 53.6 | 57.2 | 57.5 |
| (3) n-butane | 0.8 | 0.7 | 0.7 |
| (4) propane | 4.8 | 4.0 | 3.9 |
| (5) offgas | 1.5 | 1.5 | 1.6 |
| (6) water | 56.4 | 56.4 | 56.4 |
| (7) $iC_4$ | -32.1 | -32.1 | -32.0 |
| (8) methanol | -100.0 | -100.0 | -100.0 |

**Absorber Efficiency***

| | 4 | 5 | 6 |
|---|---|---|---|
| Lean Oil #1 (stage 5) g-moles/tonne Meort | 4.44 | 1.09 | 0.54 |
| Lean Oil #2 (stage 1) g-moles/ton MeOH | 1.01 | 1.01 | 1.01 |
| TOTAL | 5.45 | 2.10 | 1.55 |

| | 4 | 5 | 6 |
|---|---|---|---|
| Overhead flow, SCM/ton MEOH | 45.8 | 46.7 | 50.3 |
| propene, g-moles/ tonne MeOH | 47.7 | 37.2 | 36.4 |
| propylene | 36.1 | 97.2 | 252.1 |
| n-butane | 3.3 | 2.7 | 2.5 |
| isobutane | 62.7 | 47.7 | 39.7 |
| 1-butylene | 34.9 | 35.7 | 36.5 |
| n-pentanes | 0.6 | 0.6 | 0.6 |
| iso-pentanes | 4.9 | 5.0 | 5.0 |
| pentenes | 13.0 | 13.3 | 13.5 |
| $C_6$+ | 12.8 | 13.1 | 13.1 |
| Overhead pressure, kPa | 2068 | 2068 | 2068 |
| Cooling Pump around, MJ/tonne MeOH | -37.7 | -27.9 | -21.0 |
| Reboiler Duty, MJ/ tonne MeOH | 152.2 | 95.2 | 85.6 |

| | 4 | 5 | 6 |
|---|---|---|---|
| Ethylene recovery in overhead g-moles/tonne MEOH | 939 | 939 | 939 |
| Mole% purity | 49 | 48 | 44 |

*based on 0.2 mol% $C_2$ in bottoms

The subsequent alkylation may then be carried out in the manner described above.

The separation system shown in FIG. 6 applies also to the third process according to the invention, except that in this case, $C_3$+ components are selectively sorbed in a $C_4$+ liquid hydrocarbon sorbent stream, especially $C_4$ to $C_6$ isoparaffins.

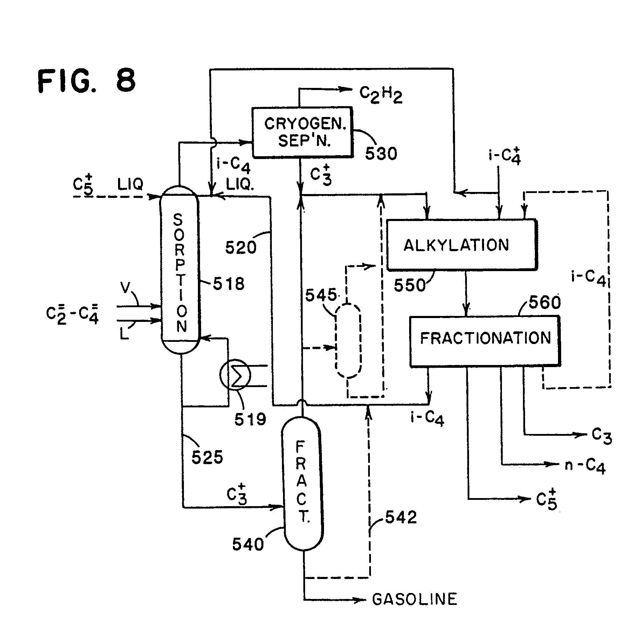

In this respect, the separation system shown in FIG. 8 is again similar to that described above with respect to FIG. 7, except that sorbent liquid, rich in $C_4$ to $C_6$ isoparaffins, is introduced via conduit 520. Optionally, other $C_4+$ liquids may be employed to supplement the alkylate stream. As before, a light olefin feedstream containing $C_2$-$C_4$ alkenes is introduced via vapor conduit V and liquid conduit L. The sorber overhead may contain 90% or more ethene, which can be further purified by cryogenic separation unit 530 to remove any remaining $C_3+$ hydrocarbons and thus to recover pure ethene. At least a portion of the i-$C_4$ component from the fractionator 560 is passed via conduit 520 to the sorption fractionator unit as lean sorbent. However, it is preferred to employ sorbent comprising a major amount of $C_4$-$C_6$ isoparaffin hydrocarbons, particularly at least 50% tertiary alkane.

The data in Table IV represents a material balance and absorber operating conditions for a countercurrent contact tower unit design according to FIG. 6 and Runs 1-3. The vertical tower has 24 theoretical stages, with $C_6+$ gasoline lean oil from the interstage sorbate fractionator being introduced at the top (stage 1), $C_5+$ heavy liquid separated from the first stage effluent being fed as a second lean oil at stage 4, isobutane (lean oil #3) being fed to stage 7, olefin vapor and liquid feed being fed at stages 14 and 15 respectively. Heat of sorption is removed by pumparound cooling at stages 5 and 8. The three runs correspond to different lean oil rates. In the overhead stream, molar units are gm-moles per metric tonne (MT) of methanol charged to the process. The operating conditions are chosen to provide a maximum ethene content of 0.2 mol% in the sorbate.

## TABLE IV

### Absorber Operation

| Run No. | 1 | 2 | 3 |
|---|---|---|---|
| **Material Balance** | | | |
| wt% of MeOH charge | | | |
| (1) $C_6+$ Gasoline | 11.5 | 11.5 | 11.5 |
| (2) Alkylate | 57.8 | 57.7 | 57.6 |
| (3) n-butane | 0.6 | 0.6 | 0.6 |
| (4) propane | 4.3 | 4.3 | 4.3 |
| (5) offgas | 1.5 | 1.5 | 1.6 |
| (6) water | 56.4 | 56.4 | 56.4 |
| (7) i-$C_4$ makeup | −32.1 | −32.0 | −32.0 |
| (8) methanol | −100.0 | −100.0 | −100.0 |
| **Absorber Operation*** | | | |
| Lean Oil #1 (stage 1) | | | |
| g-moles/tonne MeOH | 1.09 | 0.32 | 0.22 |
| Lean Oil #2 (stage 4) | | | |
| g-moles/tonne MeOH | 1.01 | 1.01 | 1.01 |
| Lean Oil #3 (stage 7) | | | |
| g-moles/tonne MeOH | 3.35 | 1.12 | 0.62 |
| TOTAL | 5.45 | 2.45 | 1.85 |
| overhead flow, SCM/tonne MeOH | 42.0 | 45.3 | 48.7 |
| propane, g-moles/ tonne MeOH | 9.2 | 23.7 | 29.5 |
| propylene | 20.1 | 106.0 | 225.6 |
| n-butane | 0.1 | 1.0 | 1.5 |
| isobutane | 3.1 | 22.8 | 31.6 |
| 1-butylene | 1.5 | 13.1 | 21.5 |
| n-pentanes | 0.3 | 0.4 | 0.4 |
| iso-pentanes | 0.4 | 0.9 | 1.4 |
| pentanes | 1.9 | 2.4 | 3.8 |
| $C_6+$ | 22.6 | 22.2 | 22.0 |
| Overhead pressure, kPa | 2068 | 2068 | 2068 |
| Cooling pump around, MJ/ tonne MeOH | −62.1 | −47.8 | −41.0 |
| Reboiler Duty MJ/tonne MeOH | 157.0 | 104.3 | 93.7 |

...

## Table IV continued

Ethylene recovery from
   overhead

| | | | |
|---|---|---|---|
| g—moles/tonne MeOH | 937 | 938 | 938 |
| Mole % purity | 53 | 49 | 46 |

\* based on 0.2 Mol% $C_2$ in bottoms

Runs 4 to 6 (Table V) are similar to Runs 1 to 3 except that the second column has 28 theoretical stages, and isobutane is introduced as the lean oil #1 only in stage 5.

TABLE V
Absorber Operation

| Run No. | 4 | 5 | 6 |
|---|---|---|---|
| **Material Balance** wt% of MeOH charge | | | |
| (1) $C_6+$ Gasoline | 11.5 | 11.5 | 11.5 |
| (2) Alkylate | 58.0 | 58.0 | 58.0 |
| (3) n-butane | 0.6 | 0.6 | 0.6 |
| (4) propane | 4.1 | 4.0 | 3.9 |
| (5) offgas | 1.5 | 1.6 | 1.6 |
| (6) water | 56.4 | 56.4 | 56.4 |
| (7) $iC_4$ makeup | -32.1 | -32.1 | -32.0 |
| (8) methanol | -100.0 | -100.0 | -100.0 |
| **Absorber Operation*** Lean Oil #1 | | | |
| g-moles/tonne MeOH | 3.34 | 1.09 | 0.54 |
| Lean Oil #2 | | | |
| g-moles/tonne MeOH | 1.01 | 1.01 | 1.01 |
| TOTAL | 4.35 | 2.10 | 1.55 |
| Overhead flow, SCM/ton MeOH | 45.7 | 46.7 | 50.2 |
| propene, g-moles/ tonne MeOH | 35.1 | 31.2 | 32.0 |
| propylene | 36.7 | 97.1 | 249.2 |
| n-butane | 3.3 | 2.7 | 2.5 |
| isobutane | 70.3 | 53.9 | 43.7 |
| i-butylene | 35.0 | 35.7 | 36.5 |
| n-pentanes | 0.6 | 0.6 | 0.6 |
| iso-pentanes | 4.9 | 5.0 | 5.0 |
| pentenes | 13.0 | 13.2 | 13.5 |
| $C_6+$ | 11.5 | 13.0 | 13.3 |
| Overhead pressure, kPa | 2068 | 2068 | 2068 |
| Cooling Pump around, MJ/tonne MeOH | -54.7 | -39.5 | -31.9 |
| Reboiler Duty, MJ/ tonne MeOH | 131.7 | 95.0 | 85.6 |
| Ethylene recovery from overhead g-moles/tonne MeOH | 938 | 939 | 939 |
| Mole % purity | 49 | 48 | 44 |

*based on 0.2 mol% $C_2$ in bottoms

As before, alkylation may then be carried out as described above.

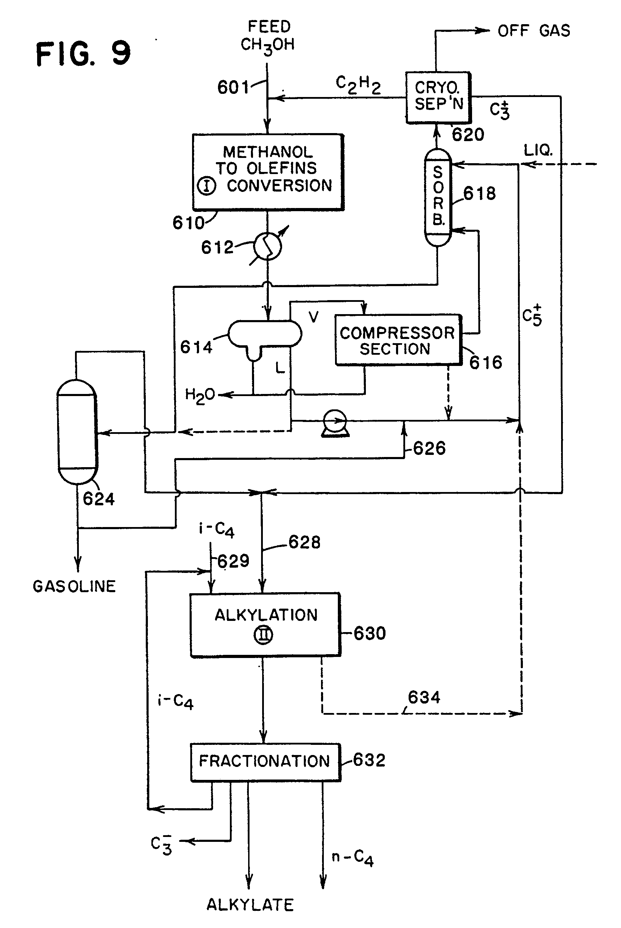

Referring now to FIG. 9, the flow scheme is again similar in many respects to that described above with reference to FIGS. 4 and 5. The primary effluent is cooled by heat exchanger 612 and byproduct water is recovered by phase separation unit 614 from the cooled effluent. Liquid hydrocarbons L consisting essentially of $C_5^+$ gasoline boiling range materials are recovered from unit 614 and pumped to the absorber unit 618 at higher (second stage) pressure. Optionally, the heavy liquid L may be sent to a fractionation system, as indicated by the broken line, to recover aromatics rich $C^+_6$ components. Alternatively, the liquid L can be prefractionated to recover $C^+_9$ aromatics as a separate stream.

Vapor phase effluent V from the primary stage may be compressed to alkylation reaction pressure in unit 616. Propylene, butylenes and amylenes may be separated from the primary stage effluent by sorption fractionation unit 618 to recover a recycle gas stream containing ethene from the primary stage and an olefinic sorbate stream rich in $C^+_3$ olefins. Purified ethene (for example, 90+%) can be obtained from cryogenic separation unit 620. A $C_3$-$C_4$ rich olefinic stream may be further prepared for reaction with isobutane or the like at high pressure and low temperature in contact with liquid phase acidic alkylation catalyst. The $C^+_3$ sorbate stream from sorber unit 618 is fractionated in tower 624 to recover $C^+_6$ aromatic and aliphatic components. A portion of the gasoline tower bottom is recycled to the sorber 618 as lean sorbent via line 626. Thus the lower olefins and other $C_2$ - $C_5$ aliphatic components are fed via conduit 628 for upgrading by reaction in the alkylation stage 630. Fresh isoparaffin (for example, i-$C_4$) and recycle are fed via conduit 629. Second stage alkylation effluent is then separated into $C^-_3$ light gases, isobutane recycle, n-$C_4$ aliphatics and $C^+_5$ gasoline and/or light distillate boiling range alkylate hydrocarbons. Advantageously, isobutane is separated from the second stage effluent for recycle to provide a stoichiometric excess and added with fresh feed (i-$C_4$) to the unit. Optionally, as indicated by broken line 634, a portion of the liquid paraffin-rich hydrocarbon may be recycled to the interstage sorption unit 618 to supplement the liquid from unit 614 as lean sorbent. The preferred sorption unit is a countercurrent packed tower. Advantageously, the sorbent liquid comprises at least 50-75 wt.% of $C^+_6$ hydrocarbons which are the first stage reaction product. This lean sorbent has excellent properties for selective sorption of the propene, butylene and $C_3$+ paraffinic components of the primary stage light hydrocarbons. Unfractionated liquid alkylation effluent may be recycled in part, as depicted by dashed line.

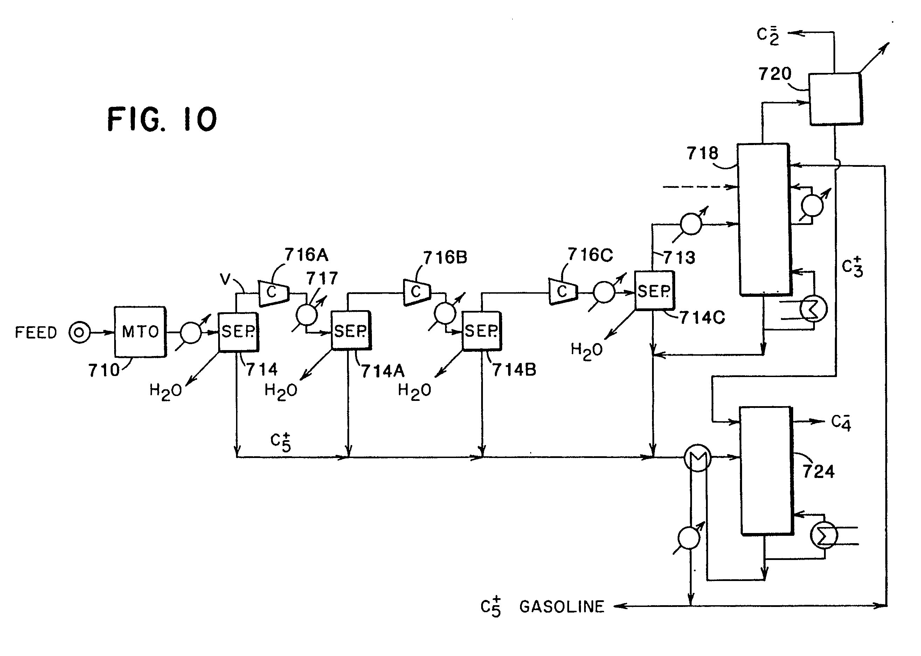

In the system of FIG. 10, oxygenate feedstock is converted in unit 710, cooled and passed to separator 714 to provide a $C_5$+ liquid stream. The light hydrocarbon vapor stream V separated from the first stage effluent is compressed in a plurality of compression stages to condense liquid olefinic hydrocarbons. The liquid stream is combined with a condensed liquid from succeeding separators. The primary vapor stream is adiabatically compressed by compressor 716A, cooled by exchanger 717 and passed to a succeeding separator 714A, at which point the preceeding phase separation technique is repeated. Similarly, a further compressor 716B, and further separators 714B and 714C operate to provide an ethene-rich recycle stream which is passed via line 713 to sorption fractionation unit 718 and optional cryogenic unit 720. Advantageously, the effluent from the unit 710 is received at about atmospheric pressure (100-150 kPa) and compressed in plural stages to a pressure of about 1500-3000 kPa and separated in the final vessel 714C at about ambient temperature (20-80°C). Olefinic liquids rich in $C^+_3$ aliphatics may be recovered from the final compressor stage and passed with $C_5$+ liquid hydrocarbon stream to fractionation tower 724 where $C_5$+ gasoline recycle and product are recovered.

A suitable selective sorption unit is that described in U.S. Patent 4,497,968. Ethene-rich vapor withdrawn from the separator 714C can be processed to increase ethene purity to at least 50% in sorption unit 718. This can be achieved by selectively absorbing $C^+_3$ components in a $C^+_5$ liquid hydrocarbon sorbent stream comprising aliphatic and aromatic hydrocarbons generated in the unit 710.

The data in Table VI represents a material balance and absorber operating conditions for a countercurrent contact tower unit design according to FIG. 10 and Runs 1-3. The vertical tower has 19 theoretical stages, with $C_6$+ gasoline lean oil from the interstage sorbate fractionator being introduced at the top (stage 1), olefin vapor and liquid feed being fed at stages 9 and 10 respectively. Heat of sorption is removed by pumparound cooling at stages 5 and 8. The three runs correspond to different lean oil rates. In the overhead stream, molar units are gm-moles per metric tonne (MT) of methanol charged to the process. The operating conditions are chosen to provide a maximum ethene content of 0.2 mol% in the sorbate.

Runs 4 to 6 (Table VII) differ from runs 1 to 3 in that two lean oil compositions are employed. To the top stage is fed gasoline sorbent from the sorbate fractionator and to a lower stage (5) is fed heavy liquid separated from the primary effluent, according to FIG. 9. Run 7 (Table VIII) is similar to run 4 except for sorbent rate and 1.5% ethene in the sorbate. Run 8 is similar to run 1 except for 0.1% ethene in the sorbate. Run 9 is similar to run 7 except for the use of compression section recontact between condensate and vapor prior to the sorption unit.

TABLE VI

| Run No. | 1 | 2 | 3 |
|---|---|---|---|
| Lean Oil* Rate (Stage 1) moles/ton MeOH charge | 5.45 | 2.00 | 1.50 |

**Material Balance**
wt% of MeOH charge

| | 1 | 2 | 3 |
|---|---|---|---|
| (1) $C_6+$ Gasoline (net) | 11.5 | 11.5 | 11.5 |
| (2) Alkylate product | 58.2 | 58.1 | 58.0 |
| (3) n-butane product | 0.6 | 0.6 | · 0.6 |
| (4) propane product | 3.9 | 3.9 | 3.9 |
| (5) offgas | 1.5 | 1.6 | 1.6 |
| (6) water byproduct | 56.4 | 56.4 | 56.4 |
| (7) i-$C_4$ makeup | −32.1 | −32.1 | −32.0 |
| (8) methanol charge | −100.0 | −100.0 | −100.0 |
| Total | 5.45 | 2.00 | 1.50 |
| overhead flow, SCM/ton MeOH | 41.0 | 44.0 | 47.6 |
| propane, moles/1000 tons MeOH | 0.0 | 5.8 | 15.9 |
| propylene | 0.8 | 111.8 | 250.0 |
| n-butane | 0.0 | 0.0 | 0.0 |
| isobutane | 0.0 | 0.0 | 0.0 |
| 1-butylene | 0.0 | 0.0 | 0.0 |
| n-pentanes | 0.2 | 0.2 | 0.3 |
| iso-pentanes | 0.4 | 0.4 | 0.4 |
| pentanes | 1.7 | 2.0 | 2.0 |
| $C_6+$ | 22.4 | 22.9 | 23.7 |
| Overhead pressure, kPa | 2068 | 2068 | 2068 |
| Cooling pump around, MJ/ton MeOH | −22.0 | −24.6 | −21.3 |
| Absorber Reboiler Duty MJ/ton MeOH | 211.4 | 117.7 | 107.1 |
| Ethene recovery in Overhead g-moles/tonne MeOH | 937 | 937 | 937 |
| Mole % | 54% | 50% | 47% |

*$C_6^+$ Gasoline

## TABLE VII

| Run No. | 4 | 5 | 6 |
|---|---|---|---|
| **Material Balance** | | | |
| wt% of MeOH charge | | | |
| (1) $C_6+$ gasoline | 11.4 | 11.4 | 11.4 |
| (2) Alkylate | 58.3 | 58.2 | 58.2 |
| (3) n-butane | 0.6 | 0.6 | 0.6 |
| (4) propane | 3.8 | 3.9 | 3.8 |
| (5) offgas | 1.6 | 1.6 | 1.7 |
| (6) water | 56.4 | 56.4 | 56.4 |
| (7) $iC_4$ | −32.1 | −32.1 | −32.1 |
| (8) methanol | −100.0 | −100.0 | −100.0 |
| **Absorber Efficiency*** | | | |
| Lean Oil 1 (stage 1) | | | |
| moles/ton MeOH | 4.44 | 1.39 | 0.74 |
| Lean Oil 2 (stage 5) | 1.01 | 1.01 | 1.01 |
| Total | 5.45 | 2.40 | 1.70 |
| Overhead flow, | | | |
| SCM/ton MeOH | 41.1 | 44.1 | 49.0 |
| propene, g-moles/ | | | |
| tonne MeOH | 0.1 | 6.3 | 20.4 |
| propylene | 1.3 | 109.0 | 292.5 |
| n-butane | 0. | 0. | 0.2 |
| isobutane | 0. | 0.2 | 1.6 |
| i-butylene | 0. | 0.8 | 6.4 |
| n-pentanes | 0.2 | 0.2 | 0.3 |
| iso-pentanes | 0.4 | 0.4 | 0.5 |
| pentenes | 1.7 | 1.9 | 2.0 |
| $C_6+$ | 15.8 | 22.9 | 23.7 |
| Overhead pressure, kPa | 2068 | 2068 | 2068 |
| Cooling Pump around, | | | |
| MJ/ton MeOH | −29.1 | −33.2 | −30.1 |
| Reboiler Duty, MJ/ | | | |
| ton MeOH | 181.0 | 104.8 | 91.9 |

*based on 0.2 mol% $C_2$ in bottoms

| Ethylene recovery in Overhead | | | |
|---|---|---|---|
| g-moles/tonne MeOH | 938 | 938 | 938 |
| Mole % purity | 54 | 50 | 45 |

Lean oil 1 -- $C_6^+$ Gasoline
Lean oil 2 -- Heavy Liquid

EP 0 216 604 B1

TABLE VIII

| Run No. | 7 | 8 | 9 |
|---|---|---|---|
| Absorber Efficiency | 1.5% $C_2$ in bottoms | 0.1% $C_2$ in bottoms | 1.5% $C_2$ in bottoms |
| Lean Oil #1* moles/ton MeOH | 1.00 | 4.00 | 1.00 |
| Lean Oil #2** moles/ton MeOH | 3.00 | ⎯⎯ | 3.00 |
| Total | 4.00 | 4.00 | 4.00 |
| Overhead flow, SCM/ ton/MeOH | 40.0 | 40.3 | 36.2 |
| propene, g-moles/ tonne MeOH | 13.0 | 2.1 | 9.9 |
| propylene | 144.4 | 48.7 | 106.6 |
| n-butane | 1.4 | 0.5 | 1.3 |
| isobutane | 5.1 | 0.3 | 4.5 |
| 1-butylene | 28.6 | 3.6 | 26.1 |
| n-pentanes | 0.8 | 1.6 | 0.7 |
| iso-pentanes | 21.4 | 47.5 | 19.9 |
| pentanes | 21.4 | 47.5 | 19.9 |
| $C_6^+$ | 9.5 | 13.2 | 8.7 |
| Overhead pressure, kPa | 2068 | 2068 | 2068 |
| Cooling Pump around MJ/tonne MeOH | -3.2 | -36.0 | -3.1 |
| Reboiler Duty, MJ/ tonne MeOH | 75.0 | 126.3 | 73.3 |

```
*   C6+ gasoline            )
                            )   both fed on stage 1
**  MTO Heavy Olefin liquid )
```

| Ethylene recovery in Overhead | | | |
|---|---|---|---|
| g-moles/tonne MeOH | 761 | 848 | 686 |
| Mole % purity | 45 | 50 | 45 |

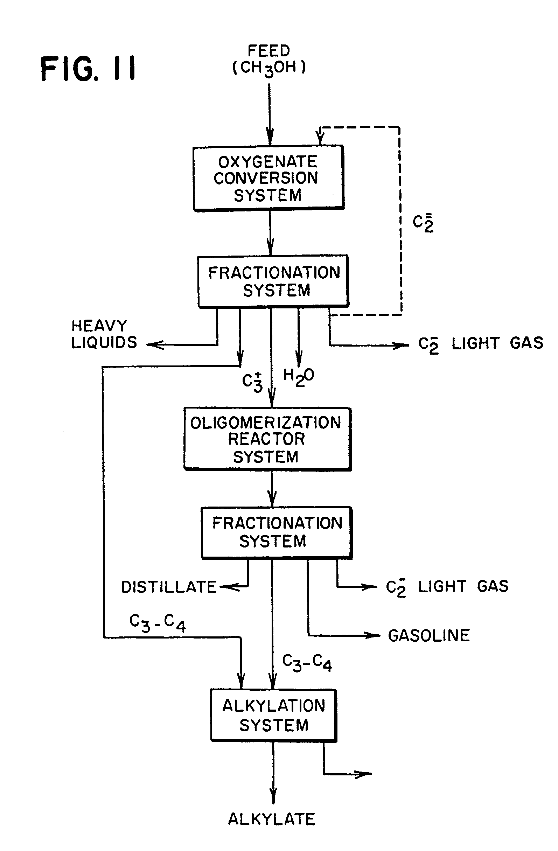

In the scheme depicted in FIG. 11, the first stage effluent is separated in a primary fractionation system to recover heavy liquid, byproduct water, ethene-rich light gas and $C_3^+$ hydrocarbons, rich in $C_3$-$C_4$ olefins. The major amount of $C_3$-$C_4$ olefins is fed to the oligomerization reactor system for upgrading to heavier hydrocarbons, especially $C_{10\ 0}^+$ distillate range aliphatics and $C_5$-$C_6$ gasoline which may be produced, fractionated and employed as described in U.S. Patent 4,497,968.

The $C_3$-$C_4$ hydrocarbon stream from the secondary oligomerization fractionation system contains unconverted propene, butylenes and isobutane ; however, the relative amounts of these components are not in stoichiometric balance for alkylation. Accordingly, a slipstream of $C_3$-$C_4$ hydrocarbons, rich in olefins, is taken from the first stage fractionation system, and bypasses the oligomerization reactor system. Thus, the alkylation reactor system receives sufficient propene and butylenes to alkylate fresh isoparaffin derived from the oligomerization reactor system.

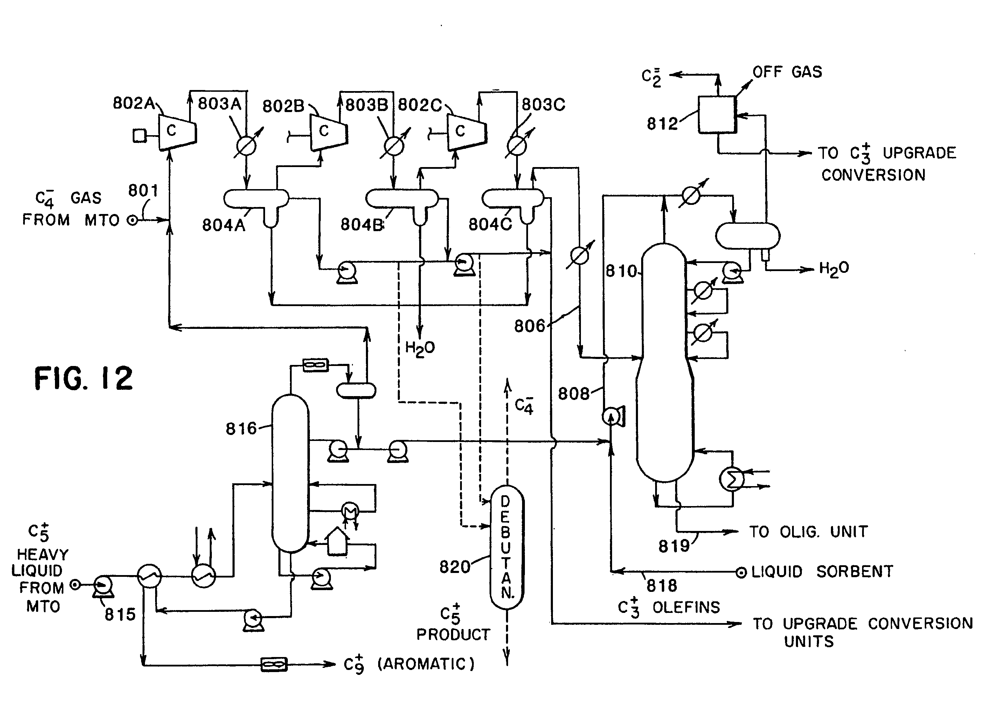

In the system depicted in FIG. 12, the first stage effluent is fractionated before being sent to olefin upgrading units. A gaseous feedstream 801 from an oxygenate-to-olefin reactor is compressed adiabatically in a series of compressors 802A, B, C and passed through corresponding coolers 803A, B, C and phase separators 804A, B, C to recover byproduct water and condensed hydrocarbons containing various amounts of $C_3$-$C_5$ aliphatics. An ethene-rich stream 806 is contacted with a liquid sorbent stream 808 in a countercurrent sorption tower 810. Ethene-rich overhead vapor from tower 810 is further purified in cryogenic sepa-

17

ration unit 812 to remove lighter gas and $C^+_3$ components. The purified ethene may be recovered or recycled to the first stage reactor for further conversion. The $C^+_3$ stream from unit 812 is rich in propene and $C_4$ aliphatics, which may be upgraded by alkylation, bypassing the oligomerization reactor.

Heavy liquid separated from the first stage (oxygenate conversion) effluent is pressurized by pump 815 and fractionated in tower 16 to recover a $C^+_9$ aromatic-rich stream. The condensed overhead, rich in $C^+_5$ aliphatic and aromatic components is combined with other liquid sorbent (for example olefinic gasoline) from line 818 and fed via line 808 to absorber unit 810. $C^+_3$ components sorbed from the feed are removed from column 810 as olefinic sorbate 819, which is a suitable feed to the oligomerization reactor for upgrading to olefinic distillate and gasoline.

As shown by the broken lines, an optional debutanizer tower 820 may be employed to recover $C^+_5$ components condensed from the compressor section. The $C^-_4$ overhead from tower 820 may be fed to either the oligomerization or alkylation reactor systems for upgrading.

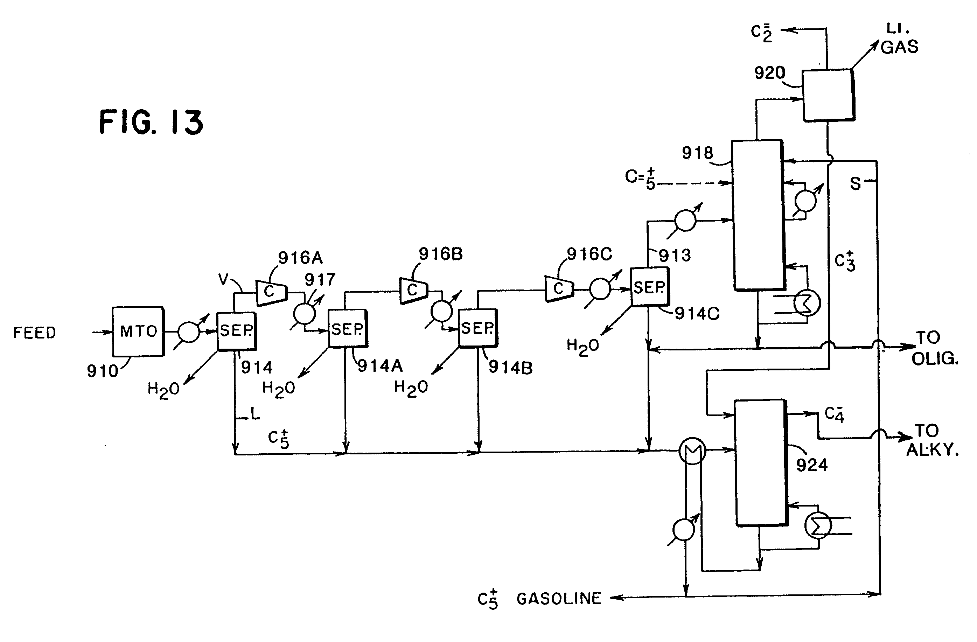

In the system of FIG. 13, oxygenate feedstock is converted in unit 910, cooled and passed to separator 914 to provide a $C^+_5$ heavy liquid stream L. The light hydrocarbon vapor stream V separated from the first stage effluent is compressed in a plurality of compression stages to condense liquid olefinic hydrocarbons. The liquid stream L is combined with a $C^+_3$-rich condensed liquid from succeeding separators. The primary vapor stream is adiabatically compressed by compressor 916A, cooled by exchanger 917 and passed to a succeeding separator 914A, at which point the preceeding phase separation technique is repeated. Similarly, other compressors 916B,C and separators 914B and 914C operate to provide an ethene-rich stream, which is passed via line 919 to sorption fractionation unit 918 and optional cryogenic unit 920. Ethene-rich vapor withdrawn from the separator 914C may be processed to increase ethene purity to at least 40% in sorption unit 918. This can be achieved by selectively absorbing $C^+_3$ components in a $C^+_5$ liquid hydrocarbon sorbent stream comprising aliphatic and aromatic hydrocarbons generated in the unit 910. Advantageously, the effluent from unit 910 is received at about atmospheric pressure (for example, 100-150 kPa) and compressed in plural stages to a pressure of about 1500-3000 kPa and separated in the final vessel 914C at about ambient temperature (20-80°C). Olefinic liquids rich in $C^+_3$ aliphatics may be recovered from the final compressor stage and passed with $C^+_5$ in the liquid hydrocarbon stream L to fractionation tower 924 where $C^+_5$ gasoline sorbent 5 and product are recovered. Olefinic gasoline ($C_5$-$C_9$) may be optionally recycled from the oligomerization stage as additional sorbent if required. A major portion of $C_3$-$C_4$ olefins may be sent to oligomerization directly from absorber 918. A suitable selective sorption unit is described in U.S. Patent 4,450,311.

## Claims

1. An integrated continuous process for converting oxygenated organic feedstock to liquid hydrocarbons comprising the steps of

(a) contacting feedstock with zeolite catalyst in a primary catalyst stage at elevated temperature and moderate pressure to convert at least a portion of the feedstock oxygenate to hydrocarbons containing a major fraction of $C_2$-$C_4$ olefins and a minor fraction containing $C_5$ + hydrocarbons ;

(b) cooling and separating effluent from step (a) to provide an aqueous liquid byproduct stream, a heavy hydrocarbon liquid stream and a light hydrocarbon vapor stream rich in $C_2$-$C_4$ olefins ;

(c) compressing at least a portion of the olefinic light hydrocarbon stream to condense a liquid olefinic hydrocarbon stream rich in $C_3$-$C_4$ olefins and recovering an ethene-rich gaseous stream ;

(d) further reacting the condensed liquid olefinic hydrocarbon stream from step (c) with isobutane in a secondary alkylation stage with acid catalyst to convert at least a portion of $C_3$-$C_4$ olefins to a heavier $C_7$+ liquid hydrocarbon product stream comprising alkylate gasoline ; and

(e) recycling ethene in a gaseous stream to the primary catalytic stage.

2. A process according to claim 1, wherein the zeolite catalyst comprises ZSM-5 type zeolite and wherein ethene is recycled to step (a) at a rate of 1 to 20 parts ethene per 100 parts by weight of methanol equivalent in the feedstock.

3. A process according to claim 1 or 2, further comprising the step of fractionating the gaseous effluent from step (b) to recover a recycle gas stream containing at least 90% ethene and a stream rich in $C_3$+ olefins.

4. A process according to any of claims 1 to 3 wherein the alkylation is catalyzed by HF.

5. A process according to any of claims 1 to 4 wherein isobutane is reacted with propene and/or butylene in the alkylation step at a pressure of 1500 to 3000 kPa.

6. A process according to any of claims 1 to 5, wherein the alkylation effluent is fractionated to provide a liquid hydrocarbon stream comprising $C_7$ to $C_9$ isoparaffins, a portion of which is recycled as lean sorbent.

**Ansprüche**

1. Integriertes kontiniuierliches Verfahren zur Umwandlung eines sauerstoffhaltigen organischen Ausgangsmaterials in flüssige Kohlenwasserstoffe, welches die Schritte umfaßt :

(a) Kontakt dieses Ausgangsmaterials mit einem Zeolithkatalysator in einer primären Katalysatorstufe bei erhöhter Temperatur und mäßigem Druck, um mindestens einen Teil der sauerstoffhaltigen Verbindungen des Ausgangsmaterials in Kohlenwasserstoffe umzuwandeln, die eine Hauptfraktion von $C_2$-$C_4$-Olefinen und eine geringere Fraktion enthalten, die $C^+_5$-Kohlenwasserstoffe enthält ;

(b) Kühlung und Abtrennung des Abflusses vom Schritt (a), um einen wässrigen flüssigen Nebenproduktstrom, einen flüssigen Strom schwerer Kohlenwasserstoffe und einen Dampfstrom leichter Kohlenwasserstoffe zu schaffen, der reich an $C_2$-$C_4$-Olefinen ist ;

(c) Komprimieren zumindest eines Teils des leichten olefinischen Kohlenwasserstoffstroms, um den flüssigen olefinischen Kohlenwasserstoffstrom zu kondensieren, der reich an $C_3$-$C_4$-Olefinen ist, und den an Ethan reichen gasförmigen Strom zu gewinnen ;

(d) weitere Reaktion des kondensierten flüssigen olefinischen Kohlenwasserstoffstroms vom Schritt (c) mit Isobutan in einer sekundären Alkylierungsstufe mit einem sauren Katalysator, um mindestens einen Teil der $C_3$-$C_4$-Olefine in einen schwereren flüssigen $C^+_7$-Kohlenwasserstoffproduktstrom umzuwandeln, der Alkylatbenzin umfaßt ; und

(e) Rezirkulierung des Ethens in einem gasförmigen Strom zur primären katalytischen Stufe.

2. Verfahren nach Anspruch 1, worin der Zeolithkatalysator einen Zeolith vom Typ ZSM-5 umfaßt, und worin das Ethen in einer Menge von 1 bis 20 Teilen Ethen pro 100 Gewichtsteilen des Methanoläquivalents im Ausgangsmaterial zum Schritt (a) rezirkuliert wird.

3. Verfahren nach Anspruch 1 oder 2, welches weiterhin den Schritt der Fraktionierung des gasförmigen Abflußes vom Schritt (b) umfaßt, um einen Umlaufgasstrom, der mindestens 90 % Ethen enthält, und einen an $C^+_3$-Olefinen reichen Strom zu gewinnen.

4. Verfahren nach einem der Ansprüche 1 bis 3, worn die Alkylierung durch HF katalysiert wird.

5. Verfahren nach einem der Ansprüche 1 bis 4, worin das Isobutan im Alkylierungsschritt bei einem Druck von 1500 bis 3000 kPa mit Propen und/oder Butylen reagiert.

6. Verfahren nach einem der Ansprüche 1 bis 5, worin der Abfluß der Alkylierung fraktioniert wird, um einen flüssigen Kohlenwasserstoffstrom zu schaffen, der $C_7$-$C_9$-Isoparaffine umfaßt, wobei ein Teil davon als schwaches Sorptionsmittel rezirkuliert wird.

**Revendications**

1. Un procédé intégré de transformation en continu de produits organiques oxygénés en hydrocarbures liquides comprenant les étapes de :

(a) mise en contact de la charge avec un catalyseur zéolitique au cours d'une étape primaire de catalyse, à température élevée et pression modérée pour transformer au moins une fraction de la charge oxygénée en hydrocarbures contenant une fraction majeure d'oléfines en $C_2$-$C_4$ et une fraction mineure contenant des hydrocarbures en $C_5$ + ;

(b) refroidissement et séparation de l'effluent de l'étape (a) pour obtenir un courant de sous-produit liquide aqueux, un courant liquide d'hydrocarbures lourds et un courant gazeux d'hydrocarbures légers riche en oléfines $C_2$ -$C_4$ ;

(c) compression d'au moins une fraction du courant d'hydrocarbures légers oléfiniques afin de condenser un courant d'hydrocarbures liquides oléfiniques riche en oléfines $C_3$-$C_4$ et récupérer un courant gazeux riche en éthène ;

(d) réaction ultérieure du courant d'hydrocarbures liquides oléfiniques condensés provenant de l'étape (c) avec de l'isobutane dans une étape secondaire d'alkylation à l'aide d'un catalyseur acide pour convertir au moins une fraction des oléfines en $C_3$-$C_4$ en un courant d'hydrocarbures liquides plus lourds $C_7$+ comprenant de l'essence alkylé ; et

(e) recyclage de l'éthène dans un courant gazeux vers l'étape primaire de catalyse.

2. Un procédé selon la revendication 1, dans lequel le catalyseur zéolitique comprend les zéolites de type ZSM-5 et dans lequel l'éthène est recyclé vers l'étape (a) à un taux de 1 à 20 parties d'éthène pour 100 parties en poids d'équivalent méthanol dans la charge.

3. Un procédé selon la revendication 1 ou 2, comprenant ultérieurement l'étape de fractionnement de l'effluent gazeux provenant de l'étape (b) afin de récupérer un courant de gaz recyclé contenant au moins 90% d'éthène et un courant riche en oléfines en $C_3$+.

4. Un procédé selon l'une des revendications 1 à 3, dans lequel l'alkylation est catalysée par HF.

5. Un procédé selon l'une des revendications 1 à 4, dans lequel on fait réagir l'isobutane avec le propène et/ou le butylène dans l'étape d'alkylation à une pression comprise entre 1 500 et 3 000 kPa.

6. Un procédé selon l'une des revendications 1 à 5, dans lequel on fractionne l'effluent d'alkylation pour obtenir un courant d'hydrocarbures liquides comprenant des isoparaffines en $C_7$ à $C_9$ dont une fraction est recyclée comme sorbant pauvre.

FIG. 1

FEED
CH₃OH

FIG. 2

FIG. 3

FIG. 4

# FIG. 5

FIG. 6

FIG. 7

# FIG. 8

# FIG. 9

FIG. 10

EP 0 216 604 B1

FIG. 11

FEED
($CH_3OH$)

OXYGENATE
CONVERSION
SYSTEM

$C_2^=$

FRACTIONATION
SYSTEM

HEAVY
LIQUIDS

$C_3^+$  $H_2O$

$C_2^-$ LIGHT GAS

OLIGOMERIZATION
REACTOR
SYSTEM

FRACTIONATION
SYSTEM

DISTILLATE

$C_2^-$ LIGHT GAS

$C_3$-$C_4$

GASOLINE

$C_3$-$C_4$

ALKYLATION
SYSTEM

ALKYLATE

FIG. 12

$C_4^-$ GAS FROM MTO

$C_5^+$ HEAVY LIQUID FROM MTO

$C_9^+$ (AROMATIC)

$C_5^+$ PRODUCT

$C_4^-$

$H_2O$

$H_2O$

$C_2^=$

OFF GAS

TO $C_3^+$ UPGRADE CONVERSION

$H_2O$

TO OLIG. UNIT

LIQUID SORBENT

$C_3^+$ OLEFINS

TO UPGRADE CONVERSION UNITS

DEBUTAN.

FIG. 13

$C_2^=$   LI. GAS

920

918

$C=_5^±$

916A   917

916B

916C   913

V

FEED   MTO   SEP.   C   SEP.   C   SEP.   C   SEP.   914C

910   914   $H_2O$   914A   $H_2O$   914B   $H_2O$

L

$C_5^±$

$C_3^+$

S

TO OLIG.

$C_4^=$

TO ALKY.

924

M

$C_5^+$ GASOLINE

32   EP 0 216 604 B1